# FASCICULE DE BREVET EUROPEEN

(11) **EP 1 332 210 B1**
(45) Date de publication et mention de la délivrance du brevet: **03.08.2011**
(21) Numéro de dépôt: 01993683.0
(22) Date de dépôt: 07.11.2001
(51) Int. Cl.: C12N 15/10

(54) **UTILISATION D'ADN POLYMERASE MUTAGENE POUR LA CREATION DE MUTATIONS ALEATOIRES**
VERWENDUNG EINER MUTAGENEN DNA-POLYMERASE ZUR HERSTELLUNG WILLKÜRLICHER MUTATIONEN
USE OF MUTAGENIC DNA POLYMERASE FOR PRODUCING RANDOM MUTATIONS

(30) Priorité: 08.11.2000 FR 0014333
(43) Date de publication de la demande: 06.08.2003
(73) Titulaire: MILLEGEN (SARL), 31525 Ramonville St. Agne (FR)
(72) Inventeur: BOUAYADI, Khalil, F-31400 Toulouse (FR); KHARRAT, Hakim, 31450 Montgiscard (FR); LOUAT, Thierry, F-42400 Saint Chamond (FR); SERVANT, Laurence, F-31400 Toulouse (FR); CAZAUX, Christophe, F-31830 Plaisance du Touch (FR); HOFFMANN, Jean, Sébastien, F-31500 Toulouse (FR)
(74) Mandataire: Touati, Catherine
(86) Numéro de dépôt international: PCT/FR2001/003450
(87) Numéro de publication internationale: WO 2002/038756

(56) Documents cités:
- WO-A-98/23733
- CADWELL R AND JOYCE G ET AL: "Randomization of genes by PCR mutagenesis" PCR METHODS AND APPLICATIONS,US,COLD SPRING HARBOR, NY, vol. 2, 1992, pages 28-33, XP002087462 ISSN: 1054-9803
- BOUAYADI K ET AL: "OVEREXPRESSION OF DNA POLYMERASE BETA SENSITIZES MAMMALIAN CELLS TO 2',3'-DEOXYCYTIDINE AND 3'-AZIDO-3'-DEOXYTHYMIDINE" CANCER RESEARCH,US,AMERICAN ASSOCIATION FOR CANCER RESEARCH, BALTIMORE, MD, vol. 57, no. 1, 1997, pages 110-116, XP002036489 ISSN: 0008-5472
- ZACCOLO M ET AL: "AN APPROACH TO RANDOM MUTAGENESIS OF DNA USING MIXTURES OF TRIPHOSPHATE DERIVATIVES OF NUCLEOSIDE ANALOGUES" JOURNAL OF MOLECULAR BIOLOGY,GB,LONDON, vol. 255, no. 4, 1996, pages 589-603, XP000612902 ISSN: 0022-2836 cité dans la demande
- ZHANG Y. ET AL: 'Human DNA polymerase kappa synthesizes DNA with extraordinarily low fidelity.' NUCLEIC ACIDS RESEARCH 1 NOV 2000 LNKD- PUBMED:11058111 vol. 28, no. 21, 01 Novembre 2000, pages 4147 - 4156 ISSN: 1362-4962
- OSHEROFF W.P. ET AL: 'Minor groove interactions at the DNA polymerase beta active site modulate single-base deletion error rates.' THE JOURNAL OF BIOLOGICAL CHEMISTRY 8 SEP 2000 LNKD- PUBMED:10851238 vol. 275, no. 36, 08 Septembre 2000, pages 28033 - 28038 ISSN: 0021-9258
- YANBIN Z. ET AL.: "Human DNA polymerase kappa synthesizes DNA with extraordinarily low fidelity." NUCLEIC ACIDS RESEARCH, vol. 28, no. 21, 1 novembre 2000 (2000-11-01), pages 4147-4156,
- OSHEROFF ET AL.: "Minor groove interactions at the DNA polymerase beta active site modulate single-base deletion error rates" THE JOURNAL OF BIOLOGICAL CHEMISTRY, vol. 275, no. 36, 8 septembre 2000 (2000-09-08), pages 28033-28038,

## Description

La présente invention concerne l'utilisation d'une ou plusieurs ADN polymérases mutagènes pour modifier des séquences d'ADN au moins partiellement codantes. Un tel procédé doit permettre l'obtention rapide d'un grand nombre de mutants aléatoires, donc des banques de polypeptides (par exemple de protéines) mutés, et permet la sélection de polypeptides (*par exemple de protéines*) d'intérêt selon un phénotype prédéterminé. Cet outil s'adresse donc en particulier à tout expérimentateur désireux de modifier une protéine soit dans un cadre d'étude fondamentale, soit dans l'optique d'une amélioration phénotypique à finalité industrielle.

Différentes techniques ont été développées pour favoriser la mutagenèse *in vitro* sur des séquences d'ADN. Parmi celles-ci, nous pouvons citer la mutagenèse dirigée et la mutagenèse aléatoire.

La mutagenèse dirigée est une méthode qui consiste à altérer *in vitro* la structure d'une protéine par simple modification de codons ciblés dans la séquence de l'ADN. Ainsi, des acides aminés peuvent être substitués au niveau de sites actifs connus ou supposés de la protéine.

La mutagenèse aléatoire *in vitro* consiste à introduire, lors d'une étape de réplication ou de recombinaison, des mutations réparties de façon aléatoire sur la séquence d'un gène ou d'un fragment de gène. Les mutations peuvent être introduites tout au long de la région codante d'un gène ou confinées à des segments d'ADN bien spécifiques. Contrairement à la mutagenèse dirigée, la connaissance précise de la structure de la protéine n'est pas nécessaire pour effectuer la mutagenèse aléatoire.

Plusieurs méthodes de mutagenèse aléatoire ont été mises au point.

Dans une première approche largement mise en oeuvre, on utilise la réaction de polymérisation en chaîne (PCR) dans des conditions qui favorisent l'introduction de mutations (error-prone PCR). Avec la *Taq* polymérase, le taux de substitution de bases peut atteindre 10⁻³ c'est-à-dire 1 substitution pour 1000 paires de bases (Moore et al. (1996) "Direct evolution of para-nitrobenzyl esterase for aqueous-organic solvant" Nat. Biotech. 14: 458-467). Dans d'autres cas, on a atteint un taux d'environ 1 substitution pour 500 bases (Sweasy et al. (1993) "Detection and characterization of mammalian DNA polymérase β mutants by functional complementation in Escherichia coli" Proc. Natl. Acad. Sci. USA 90: 4626-4630; Diaz et al. (1991) "PCR-mediated chemical mutagenesis of cloned duplex DNAs." Biotechniques 11: 204-211).

Cette technique présente cependant plusieurs inconvénients. En particulier, elle nécessite une étape de traitement du substrat à modifier par des composés chimiques génotoxiques. De plus, elle produit un taux de mutagenèse trop faible. Elle n'est donc pas adaptée pour la construction d'une banque de mutants et la sélection rapide de mutants avantageux.

Dans une autre approche, on utilise des oligonucléotides (par exemple 19 ou 47 paires de bases) de séquence aléatoire synthétisés par voie chimique (oligonucléotides dégénérés), qui sont insérés dans un gène, de préférence dans la zone codant pour la partie active de l'enzyme, et utilisés pour fournir une diversité de protéines. (Horowitz et al. (1986) "Promoters selected from random DNA sequences" Proc. Natl. Acad. Sci. USA 83: 7405-7409 ; Dube et al. (1989) "Mutants generated by the insertion of random oligonucleotides into the active site of the β-lactamase gene" Biochemistry 267: 5703-5707). Cette procédure nécessite la synthèse, encore coûteuse, de nombreux oligonucléotides mutés.

Encore une autre approche, basée sur l'utilisation de la recombinaison homologue, analogue au processus naturel de brassage génétique qui s'opère au cours de l'évolution, peut être utilisée. Cette méthode est appelée *shuffling* (Stemmer (1994) "DNA shuffling by random fragmentation and reassembly : in vitro recombination for molecular evolution" Proc. Natl. Acad. Sci. USA 91: 10747-10751). Elle consiste à faire une PCR sur des fragments d'un gène ou des fragments de plusieurs gènes homologues, à la suite d'une digestion au hasard par la DNAse I. Les petits fragments issus de cette digestion servent d'amorces les uns vis-à-vis des autres lors de la PCR, et conduisent à l'introduction de mutations aléatoires par recombinaison. Cette méthode est lourde et s'articule en plusieurs étapes (digestion de l'ADN, recombinaison par PCR en présence de « méga-amorces », etc.), ce qui implique des difficultés de mise en oeuvre. Elle a été utilisée dans l'amélioration des activités de plusieurs enzymes tels que la Green Fluorescent Protéine, la β-lactamase, ou encore l'opéron de détoxication de l'arsenate.

La fidélité de plusieurs ADN polymérases a été testée afin de mesurer leur pouvoir mutagène. Parmi celles-ci, le fragment de Klenow de l'ADN polymérase I d'*Escherichia* coli, l'ADN polymérase T4, l'ADN polymérase séquenase du phage T7, la *Taq* DNA polymérase. Parmi toute ces polymérases, la *Taq* polymérase est la moins fidèle. Cependant, le taux de mutagenèse reste trop faible pour envisager son utilisation à des fins de mutagenèse aléatoire sans modification des conditions de réaction ou de la polymérase elle-même (Cadwell et al. (1992) "Randomization of genes by PCR mutagenesis" Cold Spring Harbor Laboratory 2: 28-33).

Par conséquent, en dépit des techniques mises au point, il subsiste à ce jour un besoin pour une technique de mutagenèse aléatoire simple à mettre en oeuvre, n'impliquant pas l'utilisation de composés chimiques génotoxiques, ni d'étapes complexes, ni la synthèse de nombreux oligonucléotides, et générant un taux suffisamment élevé de mutations aléatoires pour envisager la création d'une banque de protéines.

Au terme de longues recherches, le Demandeur a démontré de façon surprenante qu'il est possible d'utiliser une mutase lors de la replication d'un ADN, afin d'obtenir un taux suffisamment élevé de mutagenèse aléatoire.

Le document WO98/23733 décrit un procédé d'identification de polymérases mutantes thermostables ayant une fidélité de réplication augmentée ou diminuée par rapport à une polymérase native. La possibilité d'utiliser de telles polymérases mutantes dont la fidélité est diminuée dans un procédé de mutagenèse aléatoire est très brièvement évoquée, mais aucune information n'est donnée, le document se focalisant sur l'identification des mutants et sur l'utilisation des mutants ayant une fidélité de réplication augmentée.

L'invention a donc pour objet un procédé de mutagenèse aléatoires tel que défini par les revendications qui comprend la replication d'une séquence d'ADN en présence d'au moins une mutase en quantité efficace. La séquence d'ADN peut être un fragment de gène ou un gène complet, qui peut coder une protéine par exemple une enzyme.

Plusieurs mutases peuvent être utilisées, soit simultanément, soit successivement. La ou les mutases utilisées seront de préférence thermostables. Les mutases utilisables peuvent être des polymérases natives, c'est-à-dire non mutées, ou des éventuellement des mutases mutées.

Par « mutase », on entend ici une ADN polymérase de niveau mutagène au moins aussi élevé que celui de l'ADN polymérase β (Pol β), en d'autres termes une ADN polymérase au moins aussi mutagène que la Pol β. Une telle mutase va introduire des nucléotides non appariés, source de mutations lors de la réplication de l'ADN, de façon aléatoire, dans un gène ou un fragment de gène.

Une mutase appropriée est Pol β, ou encore polτ, pol η, ou pol κ.

L'ADN Pol β est un petit polypeptide de 39 KD. C'est une enzyme hautement conservée chez les eucaryotes supérieurs. Sa fonction primaire serait la réparation « coûte que coûte » de l'ADN endommagé, mais elle a aussi un rôle de réplication de l'ADN natif. L'ADN Pol β est exprimée à un niveau constant au cours du cycle cellulaire, et l'exposition des cellules à des agents xénobiotiques, par exemple les rayonnements, induit son expression.

Elle n'a jamais été utilisée à ce jour en mutagenèse aléatoire. Elle se distingue des ADN polymérases habituellement utilisées en mutagenèse par son caractère infidèle lors de la réplication de l'ADN, infidélité qui serait liée à l'absence des activités exonucléasiques correctrices associées (Kunkel, T. A. (1985) "The mutational specificity of DNA polymérase-β during in vitro DNA synthesis." J. Biol. Chem. 260: 5787-5796 ; Kunkel, T. A. (1986) "Frameshift mutagenesis by eucaryotic DNA polymerases in vitro." J. Biol. Chem. 261: 13581-13587). Contrairement à ces autres polymérases utilisées, dont le caractère infidèle est insuffisant pour générer rapidement et facilement des mutations aléatoires pouvant aboutir à la création de banques de protéines mutées, le caractère infidèle de la Pol β est particulièrement élevé, et peut atteindre 10⁻².

Différentes sources de Pol β peuvent être utilisées : cellules Hela (extraits cellulaires), poulet, rat et foie humain.

On peut utiliser une Pol β native, ou une Pol β mutée.

Quelle que soit la ou les mutases utilisées, le taux de mutagenèse aléatoire est au minimum de l'ordre de 1 mutation pour 400 paires de bases, de préférence au minimum de l'ordre de 1 mutation pour 300 paires de bases, de préférence encore au minimum de l'ordre de 1 mutation pour 200 paires de bases, plus préférentiellement encore au minimum de l'ordre de 1 mutation pour 100 paires de bases, voire au minimum de l'ordre de 1 pour 50 paires de bases.

La ou les mutases utilisées seront de préférence sous forme d'extrait(s) cellulaire(s).

Le procédé selon l'invention comprend donc la réplication d'une séquence d'ADN en présence d'une quantité efficace d'au moins une mutase. L'homme de l'art saura définir cette quantité efficace. En raison des propriétés mutagènes particulières de la ou des mutases utilisées pour la réplication, on obtient des copies non fidèles de la séquence d'ADN de départ, copies portant des mutations aléatoires en nombre suffisant pour générer un ensemble de polypeptides mutés pouvant servir à créer une banque de polypeptides, et à sélectionner un ou plusieurs polypeptides présentant une ou des propriétés souhaitées. Les polypeptides peuvent être des protéines telles que des enzymes, d'origine végétale ou animale, en particulier humaine. La ou les propriétés souhaitées peuvent être par exemple une résistance à la chaleur améliorée, une meilleure efficacité, une action plus rapide ou plus ciblée, une liaison ou une meilleure liaison à un récepteur, une résistance ou une résistance améliorée à certains composés, etc.

L'étape de réplication est suivie d'une étape de recombinaison. Cette recombinaison peut être une étape de digestion d'ADN suivie par une étape d'amplification par PCR. De plus, lors de la PCR, les étapes d'hybridation et de polymérisation peuvent être regroupées en une seule étape de très courte durée. La recombinaison peut également être une étape de digestion d'ADN suivie par une étape de ligation des produits de digestion.

La PCR sert donc simplement à amplifier le matériel obtenu à l'issu de l'étape de mutagenèse. De préférence, on utilise alors des amorces d'amplification dont la longueur est telle qu'elles nécessitent une température d'amplification d'au moins 70°C, ce qui améliore la spécificité de la PCR.

Le produit de réplication, qui est porteur de mutation(s) aléatoire(s), recombiné et amplifié, est ensuite cloné dans un vecteur d'expression afin de générer les polypeptides mutés, qui sont isolés. Ces polypeptides mutés seront rassemblés en une banque de polypeptides, et pourront être sélectionnés en fonction de la ou des propriétés souhaitées.

Les propriétés mutagènes de la ou des mutases utilisées peuvent être encore accrues, afin d'augmenter encore le nombre de mutations générées. Plusieurs techniques sont possibles.

Ainsi, dans un mode de réalisation du procédé selon l'invention, on substitue du magnésium par du manganèse ou du cobalt dans la réaction de PCR (Beckman et al. (1985) "On the fidelity of DNA replication : manganese mutagenesis in vitro." Biochemistry 24: 5810-5817).

Un autre mode de réalisation du procédé selon l'invention comprend l'utilisation d'un pool de nucléotides biaisé, c'est-à-dire d'un pool de nucléotides dans lequel un ou plusieurs nucléotides sont favorisés par rapport aux autres. A titre d'exemple, on peut utiliser un pool biaisé dans lequel trois des nucléotides naturels sont favorisés par rapport au quatrième (Cadwell et al. (1992) "Randomization of genes by PCR mutagenesis." Cold Spring Harbor Laboratory 2: 28-33).

Dans un autre mode de réalisation, on incorpore un ou des analogues de nucléotides mutagènes. Un tel analogue peut être la 8-oxoguanine (Zaccolo et al. (1996) "An approch to random mutagenesis of DNA using mixture of triphosphate derivatives of nucleoside analogues" J. Mol. Biol. 255: 589-603).

On peut également utiliser une autre ADN polymérase en combinaison avec la ou les mutases, que ce soit simultanément ou successivement.

Enfin, il est possible simplement de modifier un ou des paramètres du milieu réactionnel, tels que le pH, la température ou la concentration en sels, pour modifier l'action de la ou des mutasses. Est également visé tout procédé permettant l'identification d'un composé présentant une activité ou une propriété d'intérêt, notamment dans le domaine médical, agroalimentaire ou pharmaceutique, caractérisé en ce qu'il comprend l'identification dans une banque selon l'invention d'un composé présentant ladite activité ou propriété particulière.

Dans les exemples qui suivent, il est fait référence aux figures suivantes:
- la figure 1 présente très schématiquement les étapes du procédé de production de mutations aléatoires, appliqué à un polynucléotide de 85 mers,
- les figures 2A et 2B illustrent les résultats de migrations des produits ainsi obtenus,
- la figure 3 présente le spectre de mutations ainsi engendrées par polymérisation de la matrice 85 mers,
- la figure 4 présente schématiquement la production de mutations aléatoires dans le gène *lacZ* porté par un vecteur plasmidique,
- la figure 5 présente très schématiquement les étapes d'un procédé de production de mutations aléatoires par réplication du gène *lacZ* codant pour l'α peptide de la β galactosidase, porté par le plasmide pUC18, et
- la figure 6 illustre la répartition des mutations ainsi engendrées sur le gène codant pour l'alpha peptide du gène *lacZ* dans les conditions standards de réplication avec la Pol β (tous les dNTP à 100 µM).

### EXEMPLE 1

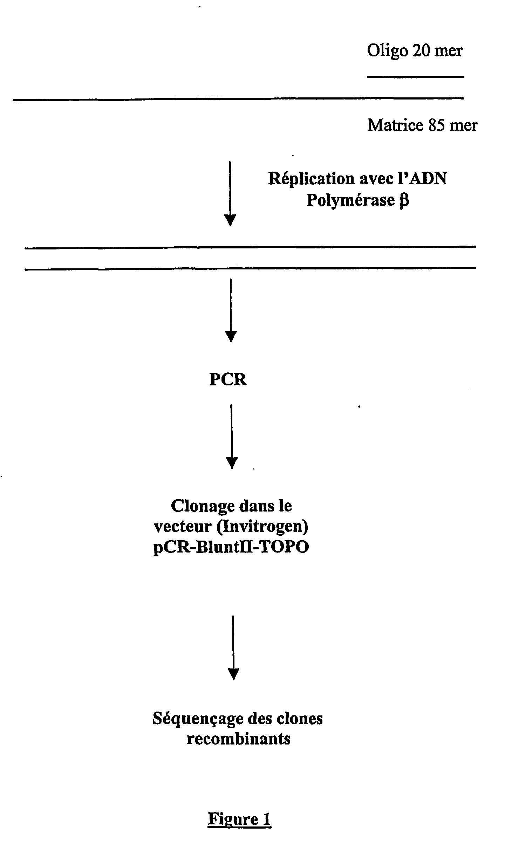

Production *in vitro* de mutations aléatoires par réplication d'un polynucléotide de 85 mers avec l'ADN Pol β et utilisation d'un pool biaisé de désoxynucléotides. La figure 1 présente très schématiquement les différentes étapes du procédé depuis le polynucléotide jusqu'au séquençage des clones recombinants.

### Matériel :

| | |
|---|---|
| Amorce 3' : | |
| 5' GATTGAATTCCTCATTATGG 3' | (SEQ ID NO: 1) |
| Amorce 5' : | |
| 5' TGAATACTGTATGATAATCG 3' | (SEQ ID NO: 2) |
| Matrice 85 mer: | |
| 5′TGAATACTGTATGATAATCGTGAGGATCCCGCATATAAGCTTTT CGATCGCCTGCAGTAACTCCACCATAATGAGGAATTCAATC 3′ | (SEQ ID NO: 3) |

### Milieux de culture

Milieu complet LB _{:} tryptone 1 %, extrait de levure 0,5 %, Nacl 1%, H₂O qsp 1.1. 15 g d'agar sont ajouté à 1 litre de LB pour la préparation du milieu LB/agar.

Milieu SOC: Tryptone 2 %, 0,5 % d'extrait de levure, 10 mM NaCl, 2,5 mM KCl, 10 mM MgCl₂, 10 mM MgSO₄, 20 mM glucose.

### Souche bactérienne et vecteurs

**TABLEAU 4 _{:}**

| Souches | Génotypes | Sources |
|---|---|---|
| TOP 10 | F⁻ *mcrA* Δ (*mrr*- *hsd*RMS-*mcr*BC) Φ80*lac*ZΔM15 Δ*lac*X74 recA1 *deo*R *ara*D139 Δ (*ara*-*leu*)7697 *gal*U *gal*K *rps*L (Str^{R}) endA1 *nup*G | Invitrogen |
| BL21 | F⁻ *ompT gal (dcm) (lon) hsdS_{B} (r_{B}⁻ m_{B}*⁻) *avec DE3,* a λ *prophage portant le gène de T7 RNA polymerase* | BioLabs |

### Replication in vitro de la matrice par l'ADN Pol β

### Préparation et purification de l'ADN Pol β

Une culture de nuit de la souche BL21 transformée par un plasmide exprimant une fusion protéique Pol β: : (His)₆ sous la dépendance d'un promoteur T7 est diluée au 1/50 dans du milieu LB+Kanamycine 50 µg/ml et incubée à 37°C sous agitation. A DO 0.6-1, de l'IPTG 1mM est ajouté et l'incubation est poursuivie pendant 4 heures. Les cellules sont centrifugées puis lysées avec du lysosyme à 100 µg/ml et du triton X100 à 0.1%. La lyse s'effectue à 30°C durant 30 min. Les cellules sont ensuite soniquées puis centrifugées. La fusion Pol β : (His)₆ est purifiée sur colonne de Nickel et élution à l'imidazole grâce à la résine et aux tampons du kit de purification Novagen.

### Marquage de l'amorce 20 mer:

60 ng de l'amorce 5' sont marqués par 10 unités de la T₄ polynucléotide kinase (New England BioLabs) pendant une heure à 37°C dans un tampon contenant 70 mM Tris-HCl (pH 7,6), 10 mM MgCl₂, 5 mM dithiotréitol et 20 µCi ³²P-γ-ATP. L'enzyme est ensuite inactivée par une incubation du mélange réactionnel à 70°C pendant 10 minutes.

### Hybridation de l'amorce marquée à la matrice

60 ng de l'amorce 5' marquée au ³²P-γ-ATP sont ajoutés à 300 ng de la matrice dans un tampon d'hybridation (100 mM Tris-HCl, 50 mM NaCl, 10 mM MgCl₂). Le mélange est d'abord dénaturé à 70°C pendant 10 minutes. L'hybridation des deux oligonucléotides est ensuite obtenue en poursuivant l'incubation jusqu'à ce que la température du bain ait atteint la température ambiante.

### Réplication de la matrice

Une unité de l'ADN Pol β est ajoutée à 5 ng de la matrice 85 mer hybridée à 20 ng d'oligonucléotide 20 mer marquée au ³²P-γ-ATP dans un mélange réactionnel contenant 25 mM HEPES (pH 8,5), 125mM NaCl, 5mM MgCl₂, 200 µM dATP, 200 µM dGTP, 200 µM dTTP. Ces différents mélanges sont répartis dans cinq tubes auxquels sont ajoutées différentes concentrations de dCTP (0 dCTP, 0.2 µM dCTP, 2 µM dCTP, 20 µM dCTP, 200 µM dCTP). Après une heure d'incubation à 37°C, une partie du mélange est déposé sur un gel d'acrylamide (15% acrylamide, 7M urée, 30% formamide) pour contrôler les produits de la réaction. L'autre partie est complémentée avec du dCTP à une concentration finale de 200 µM et la réaction est poursuivie pendant une heure. Enfin, la réaction est arrêtée avec le tampon stop (90% formamide/0,1% xylène cyanol/0,1% bleu de bromophénol/0,1 mM EDTA). Les échantillons sont ensuite dénaturés pendant 10 minutes à 70°C et chargés sur un gel d'acrylamide pour visualiser les produits de réplication. La révélation de ces produits se fait par impression d'un film autoradiographique.

### Réaction de polymérisation en chaine (PCR)

La réaction de PCR a été réalisée en ajoutant 1 µl du produit de réplication obtenue par l'action de la Pol β à un mélange contenant le tampon de polymérisation (20 mM Tris HCl (pH 8,8), 10 mM KCl, 10 mM (NH₄)₂SO₄, 0.1% Triton) (New England Biolabs) 1,5 mM MgSO4, 200 mM dNTP, 20 pmol de chaque oligonucléotide 5' et 3', et 5u de la Vent polymérase hautement fidèle (New England Biolabs). Ces mélanges ont été incubés dans un thermocycleur BioRad selon le programme suivant : (95°C, 5 min.) - (95°C 30 sec. - 50°C 30 sec. - 72°C 30 sec.) x 30 cycles - (72°C - 5 min.). Les produits de PCR sont ensuite déposés sur un gel d'agarose TAE 1%. Après migration et coloration du gel au bromure d'ethidium, la bande de 85 mer correspondant au produit d'amplification de la matrice est visualisée par trans-illumination aux ultraviolets.

### Clonage

Le clonage des produits d'amplification a été réalisé en utilisant le kit de clonage Zero Blunt TOPO PCR Cloning Kit de Invitogen. 1 µl de produit d'amplification obtenu par PCR est ajouté à 10 ng du vecteur de clonage pCR-Blunt II-TOPO (Invitrogen) contenu dans le tampon suivant : 50 % glycerol, 50 mM Tris-HCl, 1mM EDTA, 2 mM DTT, 0.1 % Triton X-100, 100 µg/ml BSA. Le mélange est maintenu à température ambiante pendant cinq minutes puis incubé à 4°C. 2 µl de la réaction de clonage sont ensuite ajoutés aux cellules compétentes Top 10 (Invitogen) et incubés pendant 30 minutes à 4°C. Un choc thermique est réalisé à 42°C pendant 30 secondes. Les cellules sont ensuite remises sur la glace avant de les incuber à 37°C pendant une heure dans 250 µl de milieu SOC. Enfin les transformants sont sélectionnés sur milieu LB/agar contenant 50 µg/ml de kanamycine.

### Extraction de l'ADN plasmidique

Les plasmides recombinants sont extraits et purifiés avec le système Quia Prep Spin de Quiagen. Les plasmides sont élués dans 50 µl d'eau stérile et contrôlés sur gel d'agarose 1% dans du TAE 1X après coloration au bromure d'ethidium et illumination par des rayonnements ultraviolets.

### Séquençage

La réaction de séquençage est réalisée avec le système SequiTherm EXCEL II DNA sequencing Kits-LC de Epicentre Technologies. 1 pmol de l'amorce M13 marquée à l'IRD700 (MWG Biotech) et 300 ng de plasmides recombinants sont incubés avec 1U de l'ADN polymérase Sequitherm EXCEL II dans le tampon de séquençage Sequitherm EXCEL II (Epicentre Technologies). Ce mélange a été incubé dans un thermocycleur PTC-100 (MJ Research, Inc.) selon le programme suivant: (95°C 5 min.) - (95°C 30 sec. - 57°C 15 sec. - 70°C 1 min.) x 30 cycles - (70°C - 10 min.). Les produits de la réaction sont ensuite déposés sur gel de séquence et analysés par le séquenceur LONG READIR 4200 (Li-COR).

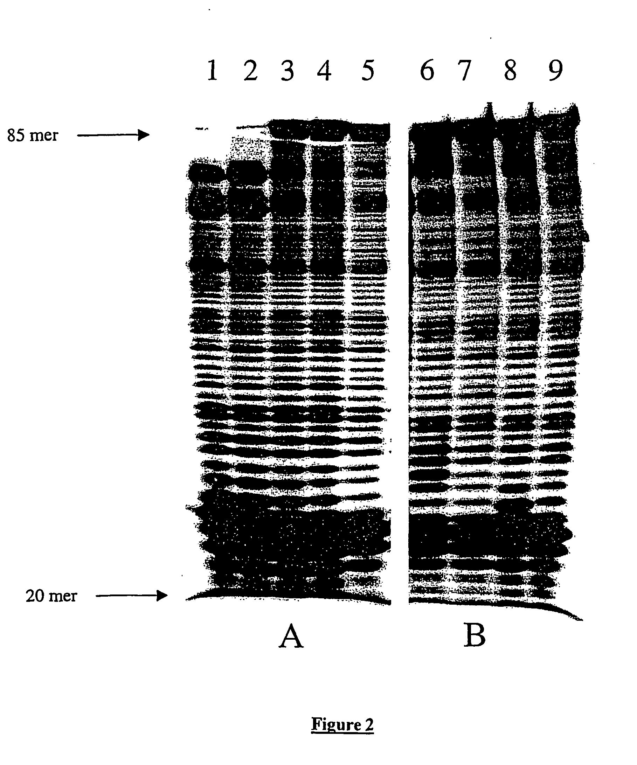

La figure 2 montre le résultat de la migration des produits de réplication par la Pol β. La partie A représente les résultats après réplication pendant une heure avec les différentes concentrations de dCTP (1 = 0 µM, 2 = 0,2 µM, 3 = 2 µM, 4 = 20 µM, 5 = 200 µM) et 200 µM de chacun des autres dNTP (dATP, dGTP, dTTP). Dans la partie B, les pistes 6, 7, 8 et 9 représentent les produits de réplication 1, 2, 3 et 4, respectivement, après complémentation de dCTP à une concentration de 200 µM et poursuite de la réaction pendant une heure.

Le Tableau 1 ci-dessous présente un bilan de la production de mutations aléatoires par l'ADN Pol β sur un oligonucléotide. Elle comprend les fréquences des mutations générées par réplication avec l'ADN Pol β dans des conditions standardisées et à partir d'un pool biaisé de désoxyribonucléotides.

**TABLEAU 1 :**

| dCTP | dATP | dGTP | dTTP | clones séquencés | Fréquences de mutations |
|---|---|---|---|---|---|
| 200µM | 200µM | 200µM | 200µM | 26 | 0.910⁻² |
| | | | | | |
| 20µM | 200µM | 200µM | 200µM | 26 | 1.2 10⁻² |
| | | | | | |
| 2µM | 200µM | 200µM | 200µM | 27 | 1.36 10⁻² |
| | | | | | |
| 0.2µM | 200µM | 200µM | 200µM | 20 | 1.7 10⁻² |
| | | | | | |
| 0µM | 200µM | 200µM | 200µM | 18 | 1.8 10⁻² |

L'expérience dans des conditions standardisées de polymérisation par la Pol β (les 4 dNTPs sont présents à une concentration de 200 µM) montre une fréquence de mutation élevée, environ une mutation tous les 100 nucléotides répliqués, confirmant ainsi le pouvoir mutagène de cette polymérase infidèle.

La Pol β est capable de répliquer entièrement la matrice de 85mers même en l'absence d'un type de nucléotide sur quatre. Ces réactions de réplication se traduisent par une augmentation du taux de mutations qui est inversement proportionnel à la concentration du quatrième nucléotide dCTP (tableau 1). Ceci confirme la potentialisation du pouvoir mutagène de la Pol β par l'association d'un biais des nucléotides dans le milieu de réaction.

Même en l'absence d'un pool biaisé de nucléotides, la fréquence des mutations observées est de 0,9 10⁻², soit près de 1 pour 100 paires de bases.

De plus, ces mutations sont réparties tout au long de la matrice, et ce de façon aléatoire. La mutagenèse n'est donc pas confinée à des sites spécifiques.

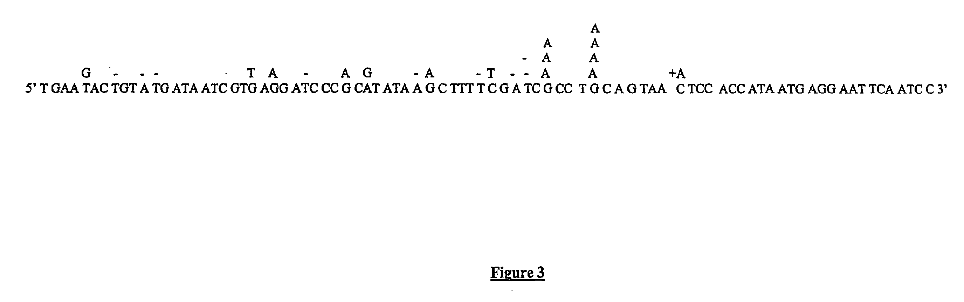

La figure 3 représente le spectre des mutations engendrées par la polymérisation de la matrice 85 mers par Pol β dans les conditions suivantes: 200 µM de dATP, dGTP, dTTP, et 0 µM de dCTP.

### EXEMPLE 2

### Production in vitro de mutations aléatoires dans le gène lacZ porté par un vecteur plasmidique.

Le but de cet essai est d'effectuer la réplication SV40 d'une matrice pBK-CMV (contenant l'origine SV40, le double gène de résistance Neo^{r}-Kan^{r} et la séquence codant pour l'α-peptide de la β-galactosidase permettant l'α-complémentation dans des souches Δ*α-lac*Z) par des extraits Hela et l'antigène T et ajouter de l'ADN Pol β purifiée de rat lors des essais. Mesurer la différence de fréquence de mutagenèse induite par l'excès de Pol β grâce au test de mutagenèse décrit ci-dessous (screen blanc/bleu).

### Souches bactériennes, plasmides, enzymes de restriction

Le plasmide pBK-CMV (Stratagène) a été amplifié chez une bactérie *dam*⁺ (DH5α) et purifié par Wizard Plus DNA Purification System (Promega). L'enzyme DpnI provient de BioLabs.

**TABLEAU 5 :**

| Souches | Génotypes | Sources |
|---|---|---|
| MC1061Mut *S* | *F⁻araD139* Δ*(araleu) 7696 galE15 galk16* Δ*lacX74 rpsL (Str^{R}) hsdR2 (rₖ⁻mk⁺) mcrA mcrB1 [muts : Tn*10] | T. Kunkel, USA |
| JM109 | *F' traD36 lacI^{q}* Δ(*lacZ) M15 proA+B+*/*e14⁻ (McrA⁻)* Δ(*lac-proAB) thi gyrA96 (NaI^{r}) endA1 hsdR17 (rₖ⁻mK⁺ ) relA1 supE44* | T. Kunkel, USA |
| DH5α. | *EndA1 hsdR17 (rₖ⁻m_{K}⁺) supE44 thi-1 recA1 gyrA (Na I^{r}) relA1* Δ *(lacIZYA-argF) U169 deoR (φ80dlacΔ(lacZ)M15)* | BioLabs |

### Préparation des extraits cellulaires réplicatifs issus de cellules Hela

La préparation des extraits cellulaires a été réalisée en suivant la procédure développée par Robert et al. (Robert et al. (1993) "Chromosome and gene analysis" Methods in Molecular Genetics.Adolph, K. W. Ed. 2: 295-313). Les cellules Hela en suspension sont cultivées dans 4 litres de RPMI1640 complémenté en sérum 9%, Pen/Strep5500, récoltées en phase exponentielle et centrifugées à 1500 rpm, 5 min, température ambiante. Elles sont lavées dans 200ml PBS 1X à 4°C et centrifugées à 1500 rpm, 5min, 4°C. Les cellules sont lavées dans 25 ml d'un milieu isotonique à 4°C (20mM Hepes-KOH pH 7.5, 5mM KC1, 1.5m MgCl₂ , 0.5mM DTT, 250mM Sucrose) et centrifugées à 4000 rpm, 10 min, 4°C. Elles sont reprises dans du milieu hypotonique à 4°C sans sucrose (20mM Hepès-KOH pH 7.5, 5mM KC1, 1.5mM MgCl₂ , 0.5mM DTT) puis centrifugées. Elles sont ensuite lysées dans un potter après ajout de 50µl d'aprotinine à 1.5mg/ml puis centrifugées à 10000 rpm, 20 min, 4°C. Le surnageant est ensuite centrifugé à 50.000 rpm, 60 min, 4°C puis aliquoté et congelé dans l'azote liquide. Le dosage de la quantité de protéines présente s'effectue selon la méthode de Bradford en utilisant la BSA pour établir la gamme étalon standard.

### Réplication SV40

Cette étape de réplication a été réalisée en utilisant l'essai développé par Robert et al. La réaction de réplication SV40 s'effectue à 37°C pendant 6h dans un volume réactionnel final de 25µl contenant 30mM HEPES-KOH pH 7,8 / 7mM MgCl₂ / 0.5mM DDT / 200µM CTP, GTP, UTP / 4mM ATP / 100µM dCTP, dGTP, dTTP / 40mM créatine phosphate / 100µg/ml créatine phosphokinase / 100ng pBK-CMV / 400µg d'extraits réplicatifs Hela / 0,5µg Large-Antigène T auquel on ajoute +/- 5µg (soit 1.2U) de Pol β. La réaction de réplication est arrêtée par l'ajout de 25µl d'un tampon contenant 2mg/ml PK, 50mM EDTA, 2% SDS et l'incubation à 55°C pendant 60 min. L'ADN est purifié par extraction au phénol, phénol/chlorophorme/alcool isoamilique, éther puis précipité par 2.5V éthanol 100%, 0.1V acétate de sodium et 1µg de glycogène et centrifugé à 13000rpm, 30min, 4°C. L'ADN est ensuite lavé à l'éthanol 70%, centrifugé (13000rpm,15 min, 4°C), et digéré par 10U de DpnI pendant 2h afin d'éliminer toute matrice pBK-CMV non répliquée au moins une fois par les extraits Hela. Il est enfin reprécipité et repris dans 10µl H₂O.

### Analyse de la mutagenèse

2µl de plasmide répliqué sont électroporés dans la souche MC1061MutS, déficiente pour le système de réparation des mésappariements, afin de fixer les mutations puis on amplifie le plasmide en sélectionnant la population électroporée par la Kanamycine (50µg/ml) sur un volume total de 20ml de LB. Le plasmide est extrait par Wizard Plus DNA Purification System (Promega) puis 100 ng est électroporé dans la souche JM109 permettant l'α-complémentation. Un volume adéquat de la population bactérienne (contenant environ 2000 à 3000 clones) est ensuite inoculé dans 7ml soft LB contenant 1.6 mg X-Gal, 1.6 mg IPTG, 350 µg Kanamycine puis étalée sur des boites de diamètre 14 contenant 40 ml de LB/agar avec 50µg/ml de Kanamycine. La fréquence de mutagenèse présente sur le plasmide répliqué est déduite du nombre de colonies blanches par rapport au nombre de colonies totales présentes sur la boîte.

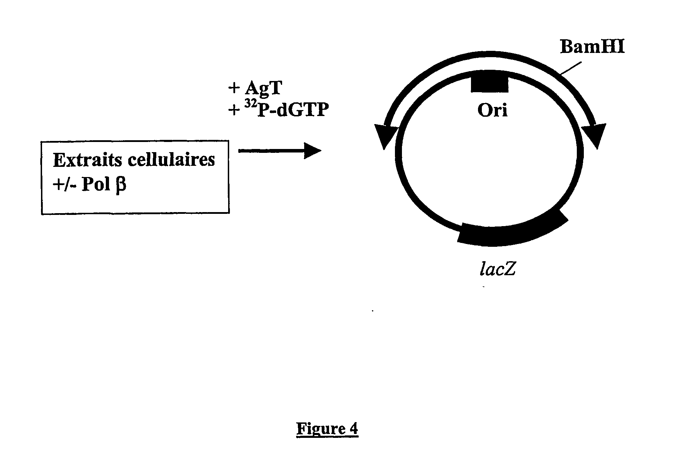

La figure 4 montre le résumé de cette production de mutations aléatoires.

Le Tableau 2 ci-dessous présente un bilan de la production de mutations aléatoires.

**TABLEAU 2 :**

| Substrat ADN | Colonies examinées | | Mutations x 10⁻³ |
|---|---|---|---|
| | Total | Mutants | |
| ADN non répliqué (pas (AgT) | 28 640 | 184 | 6,4 |
| | | | |
| ADN répliqué par des extraits cellulaires sans Pol β | 141 390 | 918 | 6,5 |
| | | | |
| ADN répliqué par des extraits cellulaires avec Pol β | 209 762 | 2 953 | 14 |

Il montre qu'en conditions physiologiques, c'est à dire en présence d'extraits cellulaires, Pol β est capable d'induire des mutations aléatoires sur une cible de mutagenèse portée par un substrat de réplication plasmidique. Encore une fois, on constate immédiatement que la fréquence des mutations induites est élevée, en l'occurrence 1,4 10⁻², soit plus de 1 pour 100 paires de bases.

### EXEMPLE 3

### Replication du gène lacZ par Pol β et comparaison avec les résultats obtenus par PCR avec une taq polymérase

Cet exemple montre que Pol β introduit un taux de mutagenèse 50 fois plus élevé que celui obtenu avec la taq polymérase par PCR.

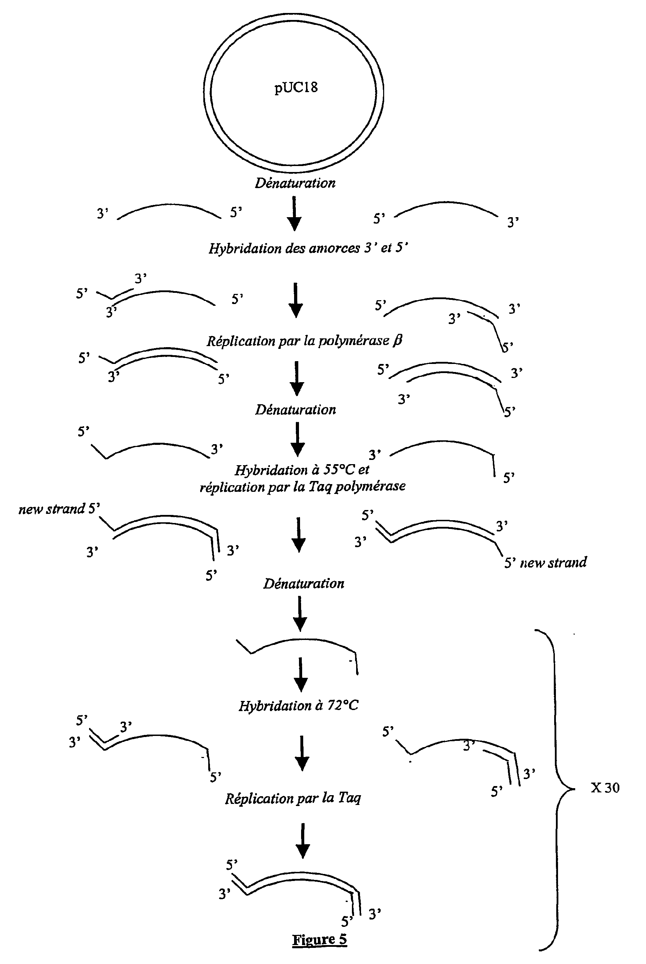

Des mutations aléatoires ont été produites *in vitro* par réplication du gène *lacZ* codant pour l'α peptide de la β galactosidase, porté par le plasmide pUC18 (invitrogen), avec l'ADN polymérase β et utilisation d'un pool biaisé de désoxynucléotides. La figure 5 présente très schématiquement les différentes étapes du procédé suivi, depuis la réplication du gène *lacZ* par la polymérase β jusqu'au séquençage des clones mutants.

### Matériel:

| | |
|---|---|
| Amorce 5' : | |
| 5' CGCGACGTCATGCGACGATTACGAATTCGAGCTCGGTAC 3' | (SEQ ID NO: 4) |
| Amorce 3' | |
| 5' CACTCGACGCTGATGCAGTGCACCATATGCGGTGTG 3' | (SEQ ID NO: 5) |

Les parties soulignées sont les séquences complémentaires des séquences en 5' et 3' flanquant le gène *lacZ* porté par le plasmide pUC18.

### Milieux de culture :

La composition des milieux est détaillée dans l'exemple 1

### Souche bactérienne :

Le génotype et l'origine de la souche Top 10 sont décrit dans l'exemple 1

### Réplication in vitro du gène lacZ par l'ADN polymérase β

1 µg de plasmide pUC18 est ajouté à 10 pmole des amorces 5' et 3' dans 15 µl de tampon de réplication ( 50 mM Tris-HCl pH 8.8, 10 mM MgC12, 100 mM KC1, 0.4 mg/ml BSA, 1 mM DTT, 10% glycérol, Trevigen). Le mélange est dénaturé pendant 5 min à 90°C puis hybridé 2 min à 55°C avant d'être conservé à 4°C. Puis une solution de 15 µl du même tampon contenant 4 unités de polymérase β (Trevigen), 0.5 mM Mn2+ et 100 µM de chacun des 4 desoxynucléotides naturels est ajouté aux 15 µl de mélange d'hybridation. La réaction de réplication par la polymérase β est ensuite réalisée à 37°C pendant une heure. Dans d'autres réactions de polymérisation, le pool des 4 désoxynucléotides est biaisé par une concentration plus faible d'un des 4 désoxynucléotides par rapport au trois autres.Les produits de réplication sont ensuite extraits au phénol-chloroforme.

### PCR sélective :

La réaction de PCR sélective est réalisée en ajoutant 1µl du produit de réplication (dilué au 1/10) obtenue par l'action de la polymérase β à un mélange contenant le tampon de PCR (20mM Tris HCl pH 8.4, 50 mM KCl) (Life Technologies), 1.5 mM MgC12, 10 pmole des amorces 5' et 3', 200 µM des 4 dNTPS et 1.25 U PLATINUM Taq DNA polymérase (Life Technologies). Ce mélange est incubé dans un thermocycleur (Eppendorf) selon le programme suivant: (95°C, 5 min.) -(94°C, 15 sec. - 55°C, 30 sec. - 72°C, 30 sec.) - ( 94°C, 15 sec. - 72°C, 30 sec.) x 30 cycles - ( 72°C, 10 min.). Ce programme permet d'amplifier spécifiquement les fragments d'ADN synthétisés par la polymérase β.
Les produits de PCR sont ensuite extraits au phénol-chloroforme, précipités à l'éthanol et récupérés dans du TE (10 mM Tris HCl pH 8, 1 mM EDTA).

### Clonage des produits de PCR :

Avant d'être clonés dans le pUC18, les produits de PCR sont digérés par 20 U de NdeI (BioLabs) et 10 U BamH1 (Q-BIOgene) dans un tampon de digestion contenant ( 50 mM NaCl, 10 mM Tris-HCl, 10 mM MgCl2, 1 mM DTT et 100 µg/ml BSA) pendant une heure à 37°C. Le produit de digestion est ensuite purifié avec le kit GenClean2 (BIO 101) après migration sur gel d'agarose et excision de la bande contenant le produit de PCR. Le produit de PCR est ensuite cloné dans le pUC18 (prédigéré par NdeI et BamH1) à la suite d'une ligation (16°C pendant une nuit) avec 40 U de T4 DNA ligase (BioLabs) dans un tampon contenant (50 mM Tris HCl pH 7.5, 10 mM MgCl2, 10 mM DTT, 1 mM ATP et 25 µg/ml BSA). Le produit de ligation est ensuite précipité à l'éthanol et repris dans 5 µl de TE (10 mM Tris HCl pH 8, 1 mM EDTA). 2 µl de la réaction de ligation sont ensuite ajoutés aux cellules compétentes Top 10 (Invitogen) et incubés pendant 30 minutes à 4°C. Un choc thermique est réalisé à 42°C pendant 30 secondes. Les cellules sont ensuite remises sur la glace avant de les incuber à 37°C pendant une heure dans 250 µl de milieu SOC. Enfin, les transformants sont sélectionnés sur milieu LB/agar contenant 100 µg/ml d'ampicilline et 60 µg/ml de X-Gal (5-bromo-4-chloro-3-indolxyl-beta-D-galactopyranoside, Euromedex). La fréquence de mutants est déduite du nombre de colonies blanches par rapport au nombre de colonies totales présentes sur la boîte.

La figure 5 montre le résumé de cette production de mutations aléatoires.

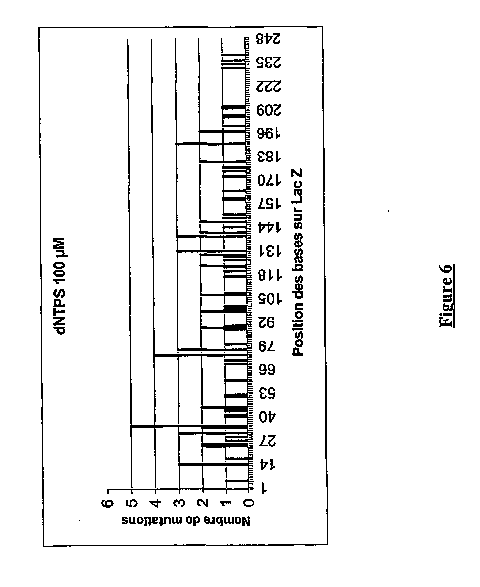

La figure 6 illustre la répartition des mutations ainsi engendrées sur le gène codant pour l'alpha peptide du gène *lac*Z dans les conditions standards de réplication avec la Pol β (tous les dNTP à 100 µM) .

Le tableau 3 ci-dessus montre un bilan de production de mutations aléatoires.

**TABLEAU 3 _{:}**

| | Clones criblés | Mutants | Fréquence de mutation (%) | Mutations/kb |
|---|---|---|---|---|
| Réplication par la Taq (dNTPS 100µM) | 2908 | 14 | **0.48** | ND |
| Réplication par Pol β (dNTPs 100µM) | 764 | 187 | **24,48** | **17,09** |
| Réplication par Pol β (dATP 100µm, dGTP 100µM, dCTP 100µM, dTTP 20µM) | 1749 | 730 | 41,74 | 18,47 |
| Réplication par Pol β (dGTP 100µM, dCTP 100µM, dTTP 100µM, dATP 20µM) | 1734 | 1078 | 62,17 | 23,6 |

Ce bilan montre que, dans les conditions normales, c'est-à-dire lorsque la réplication par la polymérase β est effectuée avec 100 µM de chacun des quatre dNTPs, la polymérase β est capable de produire des mutations aléatoires sur le gène codant pour la β galactosidase. Ce taux de mutation (24.48%) comparé à celui produit par la simple amplification par PCR par la taq polymérase (LifeTechnologies) (0.48%) est 50 fois plus élevé. Ce taux de mutation très élevé produit par la polymérase β permet le criblage d'un nombre de mutants réduit dans le but d'isoler un mutant d'une protéine dont l'activité est améliorée ou affaiblie. Ce taux de mutants est encore plus élevé lorsque la concentration d'un des quatre dNTPS est modifiée. Par exemple lorsque le dTTP ou le dATP sont utilisés séparément dans deux réactions indépendantes à une concentration de 20 µM, le taux de mutants obtenus est de 41.7% et 62.17% respectivement. Cette augmentation de taux de mutants permet de réduire encore plus le nombre de mutants à cribler.

Les résultats de ces différents essais démontrent la pertinence de l'utilisation de cette enzyme, notamment native, en vue de la création de mutants alétoires et permettent d'envisager la conception d'un outil basé sur l'utilisation de Pol β ou d'extraits cellulaires/Pol β en vue de modifier toute séquence portée par un vecteur plasmidique.

## Revendications

1. Procédé d'obtention de banques de polypeptides par mutagenèse aléatoire comprenant la réplication d'une séquence d'ADN en présence d'une quantité efficace d'au moins une mutase, **caractérisé en ce que** la mutase est une ADN polymérase β, í, η ou κ, mutée ou non mutée, et **en ce que** l'étape de réplication est suivie d'une étape d'amplification par PCR, et **en ce que** le produit de réplication amplifié est cloné dans un vecteur d'expression pour synthétiser des polypeptides mutés qui sont isolés.

2. Procédé d'obtention de polypeptides aux propriétés souhaitées par mutagenèse aléatoire comprenant la réplication d'une séquence d'ADN en présence d'une quantité efficace d'au moins une mutase, **caractérisé en ce que** la mutase est une ADN polymérase β, í, η ou K mutée ou non mutée et **en ce que** l'étape de réplication est suivie d'une étape d'amplification par PCR, et **en ce que** le produit de réplication amplifié est cloné dans un vecteur d'expression pour synthétiser des polypeptides mutés qui sont isolés, et **en ce qu'**on sélectionne les polypeptides mutés présentant la ou les propriétés souhaitées.

3. Procédé selon la revendication 1 ou 2, **caractérisé en ce que** l'on utilise la ou les mutases sous forme d'extrait (s) cellulaire (s).

4. Procédé selon l'une quelconque des revendications 1 à 3, **caractérisé en ce que** l'on utilise plusieurs mutases, simultanément ou successivement.

5. Procédé selon l'une quelconque des revendications 1 à 4, **caractérisé en ce que** l'on utilise une ADN polymérase en association avec la ou les mutases.

6. Procédé selon l'une quelconque des revendications 1 à 5, **caractérisé en ce que** le taux de mutagenèse aléatoire est au minimum de l'ordre de 1 mutation pour 400 paires de bases, de préférence au minimum de l'ordre de 1 mutation pour 300 paires de bases, de préférence encore au minimum de l'ordre de 1 mutation pour 200 paires de bases, plus préférentiellement encore au minimum de l'ordre de 1 mutation pour 100 paires de bases, voire au minimum de l'ordre de 1 pour 50 paires de bases.

7. Procédé selon l'une quelconque des revendications 1 à 6, **caractérisé en ce qu'**on utilise un pool de nucléotides biaisé et/ou un ou des analogues de nucléotides mutagènes dans l'étape de réplication.

8. Procédé selon la revendication 7, **caractérisé en ce qu'**on utilise du manganèse ou du cobalt lors de la PCR.

## Claims

1. A method of generating a library of polypeptides by random mutagenesis comprising the replication of a DNA sequence in the presence of an effective amount of at least one mutase **characterized in that** the mutase is a DNA polymerase beta, iota, eta or kappa, mutated or not mutated, and **in that** that the replication step is followed by a PCR amplification step, and **in that** the amplified replication product is cloned into an expression vector in order to synthesize mutated polypeptides which are isolated.

2. A method of generating polypeptides with desired properties by random mutagenesis comprising the replication of a DNA sequence in the presence of an effective amount of at least one mutase **characterized in that** the mutase is a DNA polymerase beta, iota, eta or kappa, mutated or not mutated, and **in that** that the replication step is followed by a PCR amplification step, and **in that** the amplified replication product is cloned into an expression vector in order to synthesize mutated polypeptides which are isolated, and **in that** the polypeptides with the desired property or properties are selected.

3. The method as claimed in claim 1 or 2, **characterized in that** the mutase(s) is (are) used in the form of (a) cell extract(s).

4. The method as claimed in any one of claims 1 to 3, **characterized in that** several mutases are used, simultaneously or successively.

5. The method as claimed in any one of claims 1 to 4, **characterized in that** a DNA polymerase is used in combination with the mutase(s).

6. The method as claimed in any one of claims 1 to 5, **characterized in that** the frequency of random mutagenesis is at least of the order of 1 mutation per 400 base pairs, preferably at least of the order of 1 mutation per 300 base pairs, more preferably at least of the order of 1 mutation per 200 base pairs, even more prefarably at least of the order of 1 mutation per 100 base pairs, or at least of the order of 1 per 50 base pairs.

7. The method as claimed in any one of claims 1 to 6, **characterized in that** a biased nucleotide pool and/or one or more mutagenic nucleotide analogs are used in the replication step.

8. The method as claimed in claim 7, **characterized in that** manganese or cobalt is used during the PCR.

## Patentansprüche

1. Verfahren zur Erstellung von Polypeptidbanken durch zufällige Mutagenese, aufweisend die Replikation einer DNA-Sequenz in Gegenwart einer wirksamen Menge mindestens einer Mutase, **dadurch gekennzeichnet, dass** die Mutase eine mutierte oder nicht-mutierte DNA-Polymerase β, í, n oder K ist, und dadurch, dass dem Replikationsschritt ein Schritt der Amplifikation durch PCR folgt, und dadurch, dass das amplifizierte Replikationsprodukt in einem Expressionsvektor kloniert wird, um mutierte Polypeptide zu synthetisieren, die isoliert werden.

2. Verfahren zur Darstellung von Polypeptiden mit erwünschten Eigenschaften durch zufällige Mutagenese, aufweisend die Replikation einer DNA-Sequenz in Gegenwart einer wirksamen Menge mindestens einer Mutase, **dadurch gekennzeichnet, dass** die Mutase eine mutierte oder nicht-mutierte DNA-Polymerase β, í, η oder K ist, und dadurch, dass dem Replikationsschritt ein Schritt der Amplifikation durch PCR folgt, und dadurch, dass das amplifizierte Replikationsprodukt in einem Expressionsvektor kloniert wird, um mutierte Polypeptide zu synthetisieren, die isoliert werden, und dadurch, dass man die mutierten Polypeptide, die die erwünschte Eigenschaft oder die erwünschten Eigenschaften aufweisen, selektioniert.

3. Verfahren nach Anspruch 1 oder 2, **dadurch gekennzeichnet, dass** man die Mutase oder die Mutasen in Form eines Zellextrakts oder von Zellextrakten verwendet.

4. Verfahren nach einem der Ansprüche 1 bis 3, **dadurch gekennzeichnet, dass** man mehrere Mutasen, gleichzeitig oder nacheinander, verwendet.

5. Verfahren nach einem der Ansprüche 1 bis 4, **dadurch gekennzeichnet, dass** man eine DNA-Polymerase in Verbindung mit der Mutase oder den Mutasen verwendet.

6. Verfahren nach einem der Ansprüche 1 bis 5, **dadurch gekennzeichnet, dass** der Grad der zufälligen Mutagenese mindestens in der Größenordnung von 1 Mutation pro 400 Basenpaaren, bevorzugt mindestens in der Größenordnung von 1 Mutation pro 300 Basenpaaren, bevorzugter mindestens in der Größenordnung von 1 Mutation pro 200 Basenpaaren, noch bevorzugter mindestens in der Größenordnung von 1 Mutation pro 100 Basenpaaren, insbesondere mindestens in der Größenordnung von 1 pro 50 Basenpaaren, liegt.

7. Verfahren nach einem der Ansprüche 1 bis 6, **dadurch gekennzeichnet, dass** man bei dem Replikationsschritt einen verzerrten Nukleotid-Pool und/oder ein Analoges oder Analoge der mutagenen Nukleotide verwendet.

8. Verfahren nach Anspruch 7, **dadurch gekennzeichnet, dass** man bei der PCR Mangan oder Kobalt verwendet.
